# FASCICULE DE BREVET EUROPEEN

(11) **EP 3 953 040 B1**
(45) Date de publication et mention de la délivrance du brevet: **30.11.2022**
(21) Numéro de dépôt: 20725778.3
(22) Date de dépôt: 31.03.2020
(51) Int. Cl.: B01L 3/00, B01D 63/08, B01D 69/10, C12M 1/00, G01N 1/40

(54) **DISPOSITIF DE DÉTERMINATION D'UNE CONTAMINATION BACTÉRIOLOGIQUE DANS UN FLUIDE**
VORRICHTUNG ZUR BESTIMMUNG DER PRÄSENZ EINER BAKTERIOLOGISCHEN KONTAMINATION IN EINEM FLUID
DEVICE FOR DETERMINING THE PRESENCE OF A BACTERIOLOGICAL CONTAMINATION IN A FLUID

(30) Priorité: 09.04.2019 FR 1903807; 21.10.2019 FR 1911739
(43) Date de publication de la demande: 16.02.2022
(73) Titulaire: BIOMERIEUX, 69280 Marcy-L'Etoile (FR)
(72) Inventeur: BUATHIER, Yoann, 69300 CALUIRE ET CUIRE (FR); HEURTAUX, Emilie, 74600 ANNECY (FR); MONTET, Marie-Pierre, 69200 VENISSIEUX (FR); ROZAND, Christine, 69290 SAINT GENIS LES OLLIERES (FR); THEVENOT, Cécile, 31860 LABARTHE SUR LEZE (FR)
(86) Numéro de dépôt international: PCT/FR2020/000076
(87) Numéro de publication internationale: WO 2020/208309

(56) Documents cités:
- WO-A1-91/18085
- CN-B- 103 432 798
- JP-A- H06 113 817
- US-A- 2 879 207
- US-A- 3 782 083
- US-A- 4 170 056

## Description

### Domaine technique de l'invention

L'invention concerne le domaine technique de l'analyse microbiologique. Plus particulièrement, l'invention porte sur un dispositif de contrôle microbiologique pour contrôler un liquide à analyser, ce liquide étant susceptible de contenir au moins un micro-organisme.

### Contexte de l'invention

L'invention peut être appliquée au domaine du contrôle microbiologique industriel, agroalimentaire, pharmaceutique, cosmétique ou vétérinaire que ce soit dans une application terrestre ou spatiale.

L'invention a été développée suite à de travaux ayant bénéficié de la participation du Centre National d'Etudes Spatiales (CNES) et de la société INITIAL.

### Arrière-plan technologique de l'invention

Il existe de nombreuses situations dans lesquelles on cherche à contrôler la présence d'au moins un micro-organisme dans un liquide, en vue généralement de pouvoir constater l'absence de ce micro-organisme. Bien entendu, le liquide à analyser peut-être un liquide biologique (sang total, sérum, plasma, urine, liquide céphalo-rachidien, sécrétion organique, etc....). Toutefois, le liquide peut aussi être un liquide industriel, notamment un liquide alimentaire (eau, boisson en général et notamment jus de fruits, lait, soda, etc..) ou un liquide pharmaceutique ou cosmétologique ou vétérinaire (lait d'animal malade).

De nombreuses techniques de laboratoire sont connues qui permettent de filtrer le liquide à analyser pour recueillir d'éventuels micro-organismes contenus dans le liquide, de mettre en culture ces micro-organismes pour être ensuite en mesure de les détecter, de les dénombrer, de les caractériser et/ou de les identifier. Ces techniques requièrent un certain nombre de manipulations et d'infrastructures (rampe de filtration, paillasses, étuves) bien connues des laborantins.

Dans ces techniques, on est souvent amené à utiliser un dispositif de filtration qui comprend un espace interne fermé délimité par une enceinte et qui est destiné à recevoir le liquide à analyser. Une telle technique est notamment appelée « filtration sur membrane ». Un moyen de filtration microbiologique, par exemple une membrane filtrante, est disposé dans l'espace interne fermé et sépare, dans l'espace interne fermé, un premier compartiment d'un second compartiment de l'espace interne fermé. Le dispositif (= cassette de filtration) comporte un port d'entrée pour le liquide à analyser qui débouche dans le premier compartiment de l'espace interne fermé.

Dans certains dispositifs de filtration connus, le dispositif est ouvert pour récupérer le moyen de filtration, lequel est transféré dans un dispositif de mise en culture pour permettre l'incubation des micro-organismes. Une telle technique est aisément réalisable en laboratoire. En revanche, une telle technique est difficilement mise en œuvre dans un milieu opérationnel, sur le terrain, au sein de la Station Internationale Spatiale et également dans un milieu industriel dans lequel les liquides sont produits, conditionnés, distribués ou utilisés.

On connait du document JPH06 113817 une capsule filtrante comprenant un filtre à membrane, un tampon absorbant et un récipient rempli d'une solution de culture staphylococcique résistante aux médicaments. Le filtre à membrane a pour fonction de collecter les bactéries en suspension dans l'air sur la surface en permettant à l'air de circuler. Le tampon absorbant est caractérisé en ce qu'après avoir collecté les bactéries avec un filtre, la solution de culture est injectée à partir du récipient rempli de solution de culture pour servir de milieu, et les staphylocoques résistants aux médicaments sont cultivés de manière sélective pour générer des colonies sur la surface de la membrane.

En outre, le document WO9118085 décrit un dispositif pour cultiver et identifier des microorganismes dans un échantillon liquide. Le dispositif comprend un récipient, un matériau poreux comprenant un nutriment sec est situé dans chaque section du compartiment du récipient, un élément filtrant poreux recouvre les matériaux poreux et piège les microorganismes.

Par ailleurs le document US2879207 décrit une unité combinée de filtration et d'incubation pour l'analyse de micro-organismes comprenant en combinaison un récipient fermé comprenant un matériau transparent permettant l'observation visuelle de l'intérieur dudit récipient et une structure de support pour un film filtrant comprenant un élément de support sous-jacent audit film filtrant.

On connait du document WO2018189478 A1, un dispositif et un procédé de contrôle microbiologique d'un liquide à analyser qui permettent des opérations de contrôle particulièrement simplifiées, qu'il serait même envisageable d'utiliser ou de conduire en dehors du laboratoire microbiologique, y compris dans un environnement industriel. Ce document décrit un dispositif de contrôle microbiologique pour contrôler un liquide à analyser susceptible de contenir au moins un micro-organisme. Le dispositif de contrôle comprend un boitier fermé destiné à recevoir le liquide à analyser, un moyen de filtration microbiologique disposé dans l'espace interne fermé et séparant, dans l'espace interne fermé, un premier compartiment d'un second compartiment de l'espace interne fermé, un port d'entrée pour le liquide à analyser débouchant dans le premier compartiment de l'espace interne fermé, une couche nutritive comprenant une composition d'un milieu de culture microbiologique. Le principal désavantage de ce dispositif est le fait que le liquide après sa filtration reste dans le dispositif et il n'est pas possible de le recycler et que Le liquide restant dans le dispositif peut lessiver le PAD, diluer le milieu nutritif et générer des faux négatifs. En outre, la fabrication du dispositif impose la mise sous vide du boitier, ce qui est une contrainte importante.

### Objet de l'invention

L'invention est une amélioration du dispositif susmentionné, plus compacte et plus pratique. À cet effet, l'invention a pour objet un dispositif de détermination d'une contamination de microorganismes d'un fluide, ledit dispositif comprenant :
- un boitier comprenant un volume interne délimité par au moins une paroi latérale et un fond,
- un couvercle fermant le boitier et positionné en regard du fond,
- un port d'entrée de fluide agencé sur le couvercle et débouchant dans le volume interne dudit boitier, le port d'entrée de fluide s'étendant selon un axe sensiblement sécant et préférentiellement perpendiculaire au couvercle,
- au moins un organe de filtration, agencé dans le volume interne,
- au moins une couche nutritive comprenant une composition d'un milieu de culture microbiologique,
caractérisé en ce que le dispositif comprend :
- un port de sortie de fluide ménagé sur le boitier et débouchant dans le volume interne dudit boitier, et en ce que le couvercle comprend une surface interne dans le volume interne s'étendant radialement autour du port d'entrée de fluide jusque sur un bord périphérique du couvercle (3), ladite surface interne étant inclinée ou incurvée et convergeant vers le port d'entrée de fluide,
- une grille de support configurée pour supporter la couche nutritive et en ce que le fond du boitier comprend une surface s'étendant radialement autour du port de sortie de fluide jusqu'à la paroi latérale du boitier, ladite surface étant inclinée et convergeant vers le port de sortie de fluide.

Avantageusement, dans le dispositif selon l'invention, le fluide traverse le dispositif et ne stagne pas dans le fond du boitier et sur le filtre, ce qui permet un passage rapide du liquide dans le dispositif avec réduction des forces requises pour ce passage et en outre d'éviter le lessivage de la couche nutritive et de s'assurer que les microorganismes seront recueillis dans les meilleurs conditions et incubés dans les meilleures conditions. De surcroit, l'inclinaison de la surface interne du couvercle permet d'améliorer la distribution du fluide dans le dispositif et de s'assurer que tout le fluide soit réparti de manière homogène sur le filtre afin de permettre une répartition homogène des micro-organismes sur le filtre, éviter ainsi la formation de tout agglomérat microbien sur le filtre ce qui pourrait générer des faux négatifs.

Avantageusement, le dispositif est configuré pour quantifier et détecter deux contaminants fécaux, par exemple E. coli et Enteroccocus bacteria.

Dans la présente invention, on entend par le terme « port » un canal de fluide présentant un orifice central traversant longitudinalement ledit canal.

Préférentiellement, le dispositif selon l'invention est utilisé verticalement c'est-à-dire que le port d'entrée de fluide et le port de sortie de fluide sont positionné verticalement.

Dans la présente demande, un objet positionné verticalement est un objet agencé selon une direction parallèle à la direction de la pesanteur.

Selon une caractéristique de l'invention, le fluide est un liquide, par exemple de l'eau ou le fluide est un gaz, par exemple de l'air.

Selon une autre caractéristique de l'invention, l'inclinaison de la surface interne du couvercle est strictement supérieure à +4°. Préférentiellement, l'inclinaison de la surface interne du couvercle est comprise préférentiellement entre +5° et +15° et encore plus préférentiellement égale à sensiblement 10°.

Selon une autre caractéristique de l'invention, l'inclinaison de la surface du fond est strictement inférieure à 0°. Préférentiellement, l'inclinaison de la surface du fond est comprise préférentiellement entre -5° et -10° et encore plus préférentiellement entre -6,5° et -7,5°.

Selon une autre caractéristique de l'invention, le port d'entrée de fluide est saillant dans le volume interne du boitier par rapport à la surface interne du couvercle.

Préférentiellement, le port d'entrée de fluide est ménagé sensiblement au centre du couvercle.

Selon une autre caractéristique de l'invention, le port d'entrée comprend une première extrémité s'étendant hors du couvercle et configurée pour coopérer avec un bouchon.

Selon une autre caractéristique de l'invention, le port d'entrée de fluide comprend une deuxième extrémité s'étendant dans le volume interne, la deuxième extrémité étant munie d'au moins un orifice latéral débouchant dans le volume interne.

Selon une autre caractéristique de l'invention, le au moins un orifice latéral est orienté pour projeter au moins partiellement le fluide vers la surface interne du couvercle.

Selon une autre caractéristique de l'invention, le port d'entrée comprend au moins deux orifices latéraux, préférentiellement agencés en regard l'un de l'autre.

Selon une autre caractéristique de l'invention, le port de sortie de fluide comprend une première extrémité s'étendant hors du boitier et configurée pour coopérer avec un bouchon.

Selon une autre caractéristique de l'invention, le port d'entrée de fluide comprend une deuxième extrémité affleurant le fond du boitier.

Selon une caractéristique de l'invention, l'organe de filtration est agencé dans le boitier à une distance déterminée h du port d'entrée. Avantageusement, la distance déterminée h est strictement supérieure à 1mm et est préférentiellement comprise entre 1,5 et 5 mm et encore plus préférentiellement entre 2,5 et 3,5mm. Il est nécessaire que l'organe de filtration soit à une distance déterminée h de manière à ce que les microorganismes, lorsque dispositif est mis en incubation puissent croitre et de former des colonies bien visibles. Cette distance déterminée conditionne un volume minimal d'air nécessaire à la croissance desdits microorganismes.

Selon une autre caractéristique de l'invention, lorsque le port d'entrée et le port de sortie de fluide sont obturés par des bouchons, le dispositif selon l'invention est étanche aux fluides.

Selon une caractéristique de l'invention, le couvercle est transparent ou translucide, ce qui permet d'observer les colonies microbiennes après incubation.

Selon une caractéristique de l'invention, le boitier de forme sensiblement cylindrique ou polygonale.

Selon une caractéristique de l'invention, le boitier comprend une première surface d'appui circonférentielle conformée pour coopérer de manière complémentaire avec un rebord du couvercle. Avantageusement, le couvercle et le boitier sont soudés ensemble par ultrason.

Selon une caractéristique de l'invention, le boitier comprend au moins une deuxième surface d'appui, s'étendant dans le volume interne et conformée pour recevoir une portion périphérique de l'organe de filtration, ladite deuxième surface d'appui étant conformée pour coopérer avec une portion du couvercle, de sorte que la portion périphérique de l'organe de filtration se trouve pincée entre le couvercle et le boitier de manière préférentiellement uniforme afin que l'organe de filtration soit stable et fixe lors du passage du fluide dans le dispositif et de de manière à s'assurer que de l'absence de fuites et que tout le fluide traverse bien l'organe de filtration.

Selon une autre caractéristique de l'invention, le fond du boitier comprend des lames de support agencées radialement par rapport au port de sortie de fluide.

Selon une caractéristique de l'invention, lesdites lames sont configurées pour soutenir la grille de support et guider le fluide vers le port de sortie de fluide.

Selon une caractéristique de l'invention, les lames de support sont saillantes par rapport à la surface du fond et s'étendent sensiblement perpendiculairement à ladite surface du fond.

Selon une caractéristique de l'invention, la pression interne au boitier est sensiblement égale à la pression externe au boitier dans des conditions terrestres, c'est-à-dire qu'aucune dépression n'est réalisée avant scellement du couvercle sur le boitier.

Selon une caractéristique de l'invention, la grille de support est configurée pour supporter la couche nutritive.

Selon une caractéristique de l'invention, la grille de support est configurée pour supporter également l'organe de filtration.

Selon une caractéristique de l'invention, la grille de support est agencée entre la couche nutritive et le fond du boitier.

Selon une caractéristique de l'invention, la grille de support est de forme globalement cylindrique ou polygonale.

Selon une caractéristique de l'invention, la grille de support comprend une pluralité d'ajours traversants répartis sur toute la grille, ce qui permet de distribuer le fluide ayant traversé la couche nutritive sur toute la surface du fond afin de mieux l'évacuer rapidement et d'éviter ainsi toute stagnation liquide dans le dispositif.

Selon une caractéristique de l'invention, la grille de support repose préférentiellement sur des lames de support ménagées dans le fond du boitier.

Selon une caractéristique de l'invention, la grille de support a préférentiellement la même taille et forme que la couche nutritive située au-dessus, de manière à éviter toute déformation de cette dernière lors du passage du liquide.

Selon une caractéristique de l'invention, la grille de support présente un périmètre conformé pour s'adapté à la forme du boitier de manière ce que la grille est maintenue dans le boitier et dans le volume interne par frottement.

Selon une caractéristique de l'invention, le boitier comprend une troisième surface d'appui 25 destinée à recevoir la grille de support et plus particulièrement à recevoir un bord périphérique de la grille de support.

Selon une autre caractéristique de l'invention, la troisième surface d'appui s'étend dans le volume interne du boitier et correspond à un épaulement externe du boitier.

Selon une caractéristique de l'invention, l'organe de filtration est perméable aux fluides et en particulier à un gaz ou à un liquide dont la viscosité permet une filtration microbiologique et débarrassé de toutes particules solides.

Selon une autre caractéristique de l'invention, l'organe de filtration est un organe de filtration imperméable aux microorganismes et plus préférentiellement aux bactéries, ce qui permet de les retenir à la surface dudit au moins un organe de filtration.

Selon une caractéristique de l'invention, l'organe de filtration est distinct de la couche nutritive. Selon une caractéristique de l'invention, l'organe de filtration est perméable aux éléments nutritifs et aux éventuels éléments additifs compris dans la couche nutritive.

Selon une caractéristique de l'invention, l'organe de filtration est réalisé sous forme d'une membrane poreuse et peut par exemple être à base de un ou plusieurs matériaux, ou dérivés de ces matériaux, parmi le latex, le polytetrafluoroethylene, le poly(vinylidene) fluoride, le polycarbonate, le polystyrène, le polyamide, le polysulphone, le polyethersulfone, la cellulose, un mélange de celluloses et nitrocellulose.

Avantageusement, l'organe de filtration présente une surface supérieure à la surface de la couche nutritive de sorte que la surface de ladite couche nutritive est entièrement recouverte par l'organe de filtration.

Préférentiellement, l'organe de filtration est de couleur blanche ou analogue, ce qui permet d'optimiser la différentiation de colonies colorées sur leur surface. Alternativement, l'organe de filtration peut être foncé pour faciliter la visibilité de colonies blanches ou crèmes.

Avantageusement, la capacité de filtration et l'hydrophilie de l'organe de filtration, quant à elles, sont mises à profit pour permettre et optimiser le passage des éléments nutritifs et des éventuels éléments additifs présents dans la couche nutritive après sa réhydratation vers une surface supérieure de l'organe de filtration tout en empêchant ou limitant la migration en sens inverse des bactéries, levures etc.. filtrées à la surface supérieure de l'organe de filtration.

Avantageusement, l'organe de filtration comporte des pores dont le diamètre est compris entre 0,01 et 0,8 microns préférentiellement entre 0,2 microns et 0,6 microns de manière à retenir les bactéries, levures et moisissures sur sa surface. Selon un mode de réalisation particulier, le moyen de filtration comporte des pores dont le diamètre est compris entre 0,25 microns et 0,6 microns, par exemple entre 0,3 microns et 0,6 microns, ou encore entre 0,4 microns et 0,6 microns. Alternativement, il peut s'agir d'une couche ne présentant pas de pores mesurables comme une membrane de dialyse. Par exemple, l'organe de filtration peut être une membrane de la marque « Fisherbrand^{™} General Filtration Membrane Filters » commercialisée par Fisher Scientific Company L.L.C, 300 Industry Drive, Pittsburgh, PA 15275, USA, ou encore une membrane de la marque « Nitocellulose Membrane Filters », fabriquée par Zefon International, Inc., 5350 SW Ist Lane, Ocala, FL 34474, USA.

Selon une caractéristique de l'invention, la couche nutritive est agencée sous l'organe de filtration et préférentiellement en contact avec ledit organe de filtration.

Selon une caractéristique de l'invention, la couche nutritive comprend un support contenant un milieu de culture microbiologique.

Selon une caractéristique de l'invention, le support peut être à base de divers composés absorbants, de préférence à très fort pouvoir de rétention d'eau, tels que la rayonne, le coton, les fibres cellulosiques naturelles ou modifiées chimiquement comme la carboxy-méthyl cellulose, les polymères chimiques absorbants ou super-absorbants tels que sels de polyacrylate, copolymère acrylate/acrylamide.

Selon une autre caractéristique de l'invention, le support peut être imprégné d'un milieu de culture microbiologique sous forme liquide.

Avantageusement, le milieu de culture microbiologique peut être avantageusement déshydraté, c'est à dire ayant une « Aw » (Activity of water, Activité en eau) incompatible avec le développement microbien. Alternativement, le support peut être recouvert ou imprégné à sec d'un milieu de culture microbiologique ou de ses constituants sous forme de poudre. Alternativement, l'imprégnation liquide peut, après déshydratation, être complétée par ajout de poudre.

On entend par milieu de culture microbiologique, un milieu comprenant des éléments nutritifs nécessaires à la survie et/ou à la croissance de microorganismes, notamment un ou plusieurs parmi les hydrates de carbone, dont les sucres, les peptones, les facteurs de croissances, les sels minéraux et/ou les vitamines, etc... En pratique, l'homme du métier choisira le milieu de culture microbiologique en fonction des micro-organismes cibles, selon des critères parfaitement connus et à la portée de cet homme de l'art. La couche nutritive peut contenir d'éventuels éléments additifs comme par exemple :
- un ou plusieurs agents sélectifs tels que des inhibiteurs ou des antibiotiques pour favoriser la croissance et le développement d'une espèce/souche de micro-organisme particulier plutôt qu'une autre ;
- des tampons, colorants.

D'une manière générale, la couche nutritive peut en sus contenir un substrat permettant la détection d'une activité enzymatique ou métabolique des micro-organismes cibles grâce à un signal détectable directement ou indirectement. Pour une détection directe, ce substrat peut être lié à une partie faisant office de marqueur, fluorescent ou chromogène. Pour une détection indirecte, la couche nutritive selon l'invention peut comporter en sus un indicateur de pH, sensible à la variation de pH induite par la consommation du substrat et révélant la croissance des micro-organismes cibles. Ledit indicateur de pH peut être un chromophore ou un fluorophore. On citera comme exemples de chromophores le rouge neutre, le bleu d'aniline, le bleu de bromocresol. Les fluorophores comprennent par exemple la 4-méthylumbelliferone, les dérivés de l'hydroxycoumarine ou les dérivés de la résorufine. Ainsi, le substrat fluorescent de PC-PLC préférentiellement utilisé pour la mise en œuvre du procédé selon l'invention correspond au 4-Méthyl- Umbelliferyl-Choline Phosphate (4 MU-CP).

Selon une caractéristique de l'invention, le milieu de culture microbiologique de la couche nutritive est, dans une configuration de mise à disposition du dispositif selon l'invention avant utilisation, déshydraté. Dans ce cas, après imprégnation à sec du support de la couche nutritive par le milieu de culture microbiologique déshydraté, la couche nutritive peut faire l'objet d'une opération de calandrage. Le calandrage, par la pression et la température de chauffe générées, permet la rétention et le maintien stable dans le temps du milieu de culture microbiologique déshydraté dans le support de la couche nutritive, en assurant la rétention des éléments nutritifs et des éventuels éléments additifs dans la couche nutritive. Le calandrage de la couche nutritive permet également l'obtention d'une surface lisse et plane de la couche nutritive. Le calandrage permet en outre l'accélération de la réhydratation de la couche nutritive par rapport à une couche nutritive non-calandrée, du fait de la compression de la couche nutritive qu'il induit. Dans le cas où le support est formé de fibres, cette compression, associée à la présence du milieu déshydraté au sein de la couche nutritive, génère une forte augmentation du pouvoir capillaire de cette dernière, provoquant sa réhydratation quasi- instantanée. Cela peut aussi contribuer à un phénomène d'aspiration de l'organe de filtration microbiologique distinct disposé contre sa surface. L'organe de filtration microbiologique peut ainsi se retrouver plaqué contre la couche nutritive, assurant ainsi l'absence d'espace ou la réduction d'espace entre les deux, au profit d'une croissance et/ou survie microbienne optimale(s) sur toute la surface du moyen de filtration microbiologique. On peut ainsi éviter la présence de moyen de liaison (par exemple éviter la présence de couche liante) entre l'organe de filtration microbiologique et la couche nutritive quand sont ceux-ci sont distincts. Ceci représente un avantage significatif, dans la mesure où un tel moyen de liaison ralentirait le passage des éléments nutritifs et des éventuels éléments additifs de la couche nutritive réhydratée vers les micro-organismes présents sur le moyen de filtration microbiologique, réduisant ainsi la croissance et/ou les chances de survie de ces micro-organismes.

Préférentiellement, la couche nutritive peut être du type du dispositif de culture microbiologique décrit dans la demande de brevet FR19/03751 déposée le 8 avril 2019.

Selon une caractéristique de l'invention, la couche nutritive est un dispositif de culture microbiologique comprenant tout ou partie d'un milieu de culture microbiologique déshydratée sous forme de poudre, comprenant au moins deux pièces en matériau hydrophile et absorbant ayant une face supérieure au moins sensiblement plane, et comprenant entre deux pièces contigües ledit milieu de culture microbiologique déshydratée sous forme de poudre, et ledit milieu de culture microbiologique comprenant au moins un agent gélifiant sous forme de poudre.

De nombreux matériaux absorbants, hydrophiles et non hydrosolubles, peuvent être utilisés pour réaliser la pièce en matériau absorbant d'un dispositif de culture microbiologique selon l'invention. Ces matériaux sont principalement choisis pour leur pouvoir absorbant, leur capacité de rétention des liquides aqueux et leur faculté à laisser les liquides aqueux les traverser dans tous les sens.

Selon une caractéristique de l'invention, la pièce en matériau absorbant est réalisée à partir d'un substrat de fibres courtes non-tissés, constituant un ensemble ayant une intégrité structurelle et une cohérence mécanique. Les substrats particulièrement adaptés sont en fibre cellulosique naturelle (comme le coton) ou synthétique (comme la rayonne), en fibre cellulosique modifiée (par exemple, la carboxyméthylcellulose, la nitrocellulose), en fibre de polymères chimiques absorbants (tels que les sels de polyacrylate, les copolymères acrylate/acrylamide). Avantageusement, la pièce en matériau absorbant est en textile non-tissé réalisé en fibres de cellulose.

Dans le cadre de la présente invention, les pièces en matériau hydrophile et absorbant d'un même dispositif peuvent avoir la même masse volumique ou avoir une masse volumique différente. De même, les pièces en matériau hydrophile et absorbant d'un même dispositif de culture microbiologique selon l'invention peuvent présenter une épaisseur identique ou différente.

Selon une caractéristique de l'invention, la pièce en matériau hydrophile et absorbant présente une masse volumique comprise entre comprise entre 0,045 g.cm-3 et 0,10 g.cm-3, préférentiellement entre 0,05 g.cm-3 et 0,07 g.cm-3.

Les pièces en matériau hydrophile et absorbant d'un même dispositif de culture microbiologique selon l'invention peuvent présenter une épaisseur comprise entre 0,5 mm et 2mm. Préférentiellement, la pièce en matériau hydrophile et absorbant a une épaisseur de 0,8 mm à 1,8mm, encore préférentiellement de 1 à 1,5mm. La surface de la pièce en matériau hydrophile et absorbant est comprise entre 1 cm² et 40 cm² préférentiellement entre 10 cm² et 30 cm², plus préférentiellement entre 15 cm² et 25 cm².

Selon une caractéristique de l'invention, la pièce en matériau hydrophile et absorbant est apte à retenir un volume d'eau supérieur à 2ml, préférentiellement supérieur à 3ml. Ainsi, un support poreux de 25 cm² de surface et une épaisseur de 1mm après calandrage aura la capacité à retenir un volume d'eau de 3ml.

Selon une caractéristique de l'invention, le dispositif de culture microbiologique a subi une opération de calandrage. Le calandrage, par la pression et la température de chauffe générées, permet la rétention et le maintien stable dans le temps du milieu réactionnel déshydraté comprenant le ou les agents gélifiants dans le dispositif de culture microbiologique, en assurant la rétention des différents éléments tels que les éléments nutritifs entre les pièces en matériau hydrophile et absorbant.

Préférentiellement le calandrage se fait à une température supérieure à la température ambiante, préférentiellement à une température comprise entre 30°C et 60°C. Une température inférieure à 60°C permet de ne pas dénaturer les composés thermolabiles du milieu de culture.

Le calandrage permet ainsi de maintenir le milieu réactionnel à l'intérieur du dispositif de culture microbiologique et rend sa manipulation aisée.

Selon une autre caractéristique de l'invention, le milieu de culture comprend en outre au moins un agent gélifiant également sous forme de poudre. Une fois activé, réhydraté, par un liquide à analyser, le ou les agents gélifiants vont permettre de créer un ensemble présentant une certaine intégrité structurelle et cohérence mécanique. De plus, du fait de leur présence au contact du milieu de culture, le ou les agents gélifiants vont donner au dispositif de culture microbiologique une consistance gélosée favorisant ainsi l'implantation des microorganismes et vont également permettre aux éléments constituants le milieu de culture, tels que les ingrédients nutritifs ou les agents actifs, de se retrouver au plus près des microorganismes.

Selon une autre caractéristique de l'invention, l'agent gélifiant est choisi parmi : une gomme de xanthane, l'alginate, une gomme de gellane, une gomme de galactomannane, de gomme de graine de caroube, l'amidon, et un mélange de ceux-ci.

Préférentiellement, le milieu de culture du dispositif selon l'invention comprend au moins un gélifiant dont la quantité est comprise entre 0.0030 g.cm-3 et 0.020 g.cm-3.

L'invention a également pour objet un ensemble comprenant au moins un au moins une amenée de fluide, et au moins un dispositif selon l'invention, ledit dispositif étant raccordé par son port d'entrée de fluide à une amenée de fluide.

Avantageusement, l'ensemble comprend en outre un deuxième dispositif selon l'invention monté en parallèle du premier dispositif selon l'invention.

Selon une autre caractéristique de l'invention, l'ensemble comprend une pluralité de dispositifs selon l'invention montés en parallèle les uns des autres.

Avantageusement, les dispositifs de la pluralité peuvent être identiques ou bien être dédiés à un type de microorganisme différent.

Selon une autre caractéristique de l'invention, le port de sortie de fluide peut être raccordé à un récipient ou non.

Selon une caractéristique de l'invention, le ou les dispositifs selon l'invention peuvent être logés dans des compartiments au niveau d'une structure de canalisations industrielles ou autre.

Selon une caractéristique de l'invention, le dispositif a une capacité volumique permettant l'analyse de 100ml de liquide.

### Brève description des figures

L'invention sera mieux comprise, grâce à la description ci-après, qui se rapporte à un mode de réalisation selon la présente invention, donné à titre d'exemple non limitatif et expliqué avec référence aux figures schématiques annexées. Les figures schématiques annexées sont listées ci-dessous :
[Fig. 1] est une vue en perspective du dispositif de détermination de contamination selon l'invention,
[Fig. 2] est une vue en coupe transversale médiane du dispositif représenté en figure 1,
[Fig. 3] est une vue de détail de la figure 2,
[Fig. 4] est une vue en perspective partielle du dispositif selon l'invention illustrant la grille de support logée dans le boitier,
[Fig. 5] est une vue en perspective de dessous du couvercle du dispositif selon l'invention,
[Fig. 6] est une vue en perspective de l'intérieur du boitier du dispositif selon l'invention,
[Fig. 7] est une vue en coupe transversale du couvercle et de l'organe de filtration du dispositif selon l'invention, et,
[Fig. 8] est une vue en coupe transversale du boitier du dispositif selon l'invention.
[Fig. 9] est une vue schématique d'une première étape d'un premier mode d'utilisation du dispositif selon l'invention,
[Fig. 10] est une vue schématique d'une deuxième étape du premier mode d'utilisation du dispositif selon l'invention,
[Fig. 11] est une vue schématique d'une troisième étape du premier mode d'utilisation du dispositif selon l'invention,
[Fig. 12] est une vue schématique d'une première étape d'un deuxième mode d'utilisation du dispositif selon l'invention,
[Fig. 13] est une vue schématique d'une deuxième étape du deuxième mode d'utilisation du dispositif selon l'invention,
[Fig. 14] est une vue schématique d'une troisième étape du deuxième mode d'utilisation du dispositif selon l'invention.

### Description détaillée des figures

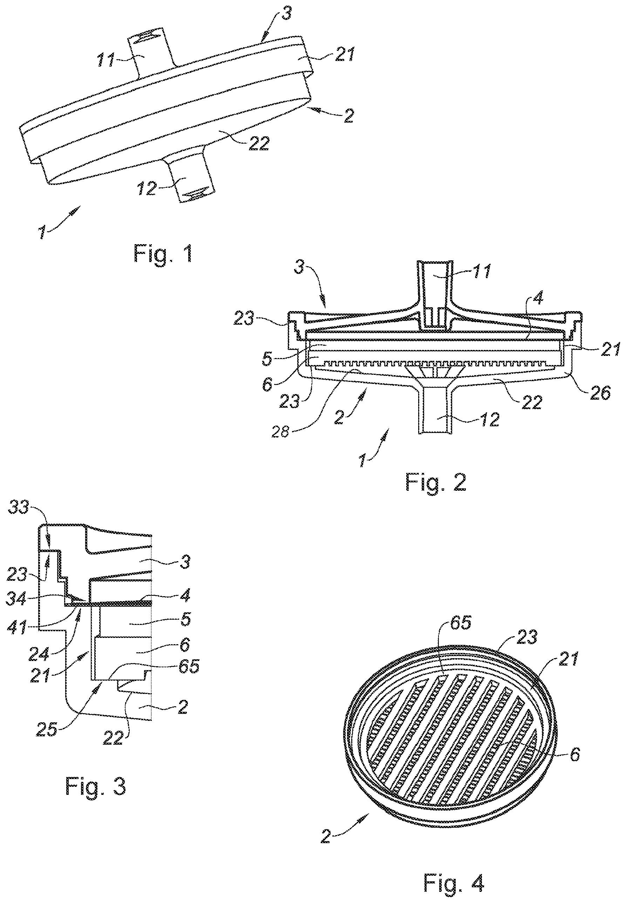

Le dispositif 1 de détermination d'une contamination de microorganismes d'un fluide selon l'invention comprend un boitier 2, un couvercle 3, un organe de filtration 4, une couche nutritive 5 et une grille 6, comme illustré notamment en figure 2.

Comme on peut le voir en figure 1, seuls le couvercle 3 et le boitier 2 sont visibles depuis l'extérieur. Avantageusement, le couvercle 3 et le boitier 2 sont scellés ensemble par ultrason. Selon la coupe transversale représentée en figure 2, on peut voir que l'organe de filtration 4 est agencé dans le volume interne du boitier 2, la couche nutritive 5 est agencée sous l'organe de filtration 4, préférentiellement en contact avec ce dernier et une grille de support 6 est agencée entre la couche nutritive 5 et le fond 22 du boitier 2.

Selon l'invention, l'organe de filtration 4 est perméable aux fluides et en particulier à un gaz ou à un liquide dont la viscosité permet une filtration microbiologique et débarrassé de toutes particules solides.

Le dispositif 1 comprend en outre un port d'entrée de fluide 11 et un port de sortie de fluide 12 comme illustré en figure 1. Le port d'entrée de fluide 11 est positionné sur le couvercle 3 et le port de sortie de fluide 12 est positionné sur le boitier 2 et préférentiellement sur le fond du boitier 2. Le port d'entrée de fluide 11 et le port de sortie de fluide 12 débouchent dans le volume interne du boitier 2, comme visible en figure 2. Lorsque le port d'entrée et le port de sortie de fluide sont obturés par des bouchons, le dispositif selon l'invention est étanche aux fluides.

Comme on peut le voir en figure 2 par exemple, le port d'entrée de fluide 11 est saillant dans le volume interne du boitier 2 par rapport à la surface interne 31 du couvercle 3. Préférentiellement, le port d'entrée de fluide 11 est ménagé sensiblement au centre du couvercle 3. Le port d'entrée de fluide comprend une première extrémité 11a s'étendant hors du couvercle 3 et configurée pour coopérer avec un bouchon (non représenté) et une deuxième extrémité 11b s'étendant dans le volume interne, la deuxième extrémité étant munie d'au moins un orifice latéral 11c débouchant dans le volume interne et orienté pour projeter au moins partiellement le fluide vers la surface interne 31 du couvercle 3.

Comme on peut le voir en figure 2 par exemple, le port de sortie de fluide 12 comprend une première extrémité 12a s'étendant hors du boitier 2 et configurée pour coopérer avec un bouchon et une deuxième extrémité 12b affleurant le fond 22 du boitier 2.

Le port d'entrée de fluide 11 et le port de sortie de fluide 12 s'étendent respectivement selon un axe sensiblement sécant et préférentiellement perpendiculaire au couvercle 3 ou au fond du boitier 2. Dans l'exemple illustré en figure 2, le port d'entrée e fluide 11 et le port de sortie de fluide 12 sont alignés et en regard l'un de l'autre.

Le boitier 2 du dispositif 1 va maintenant être décrit en détail en référence aux figures 1, 2, 6 et 8. Comme on peut le voir en figure 1, le boitier 2 de forme sensiblement cylindrique.

Comme illustré en figure 2, le boitier 2 comprend un volume interne délimité par au moins une paroi latérale 21 et un fond 22.

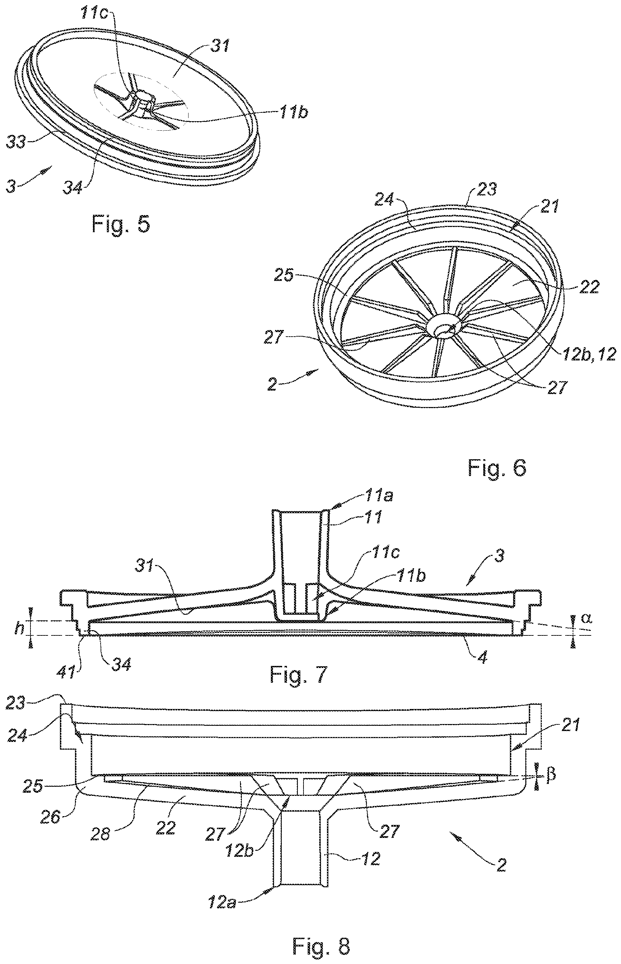

En figure 2 et 8, le boitier 2 est illustré en coupe transversale ce qui permet de voir une première surface d'appui circonférentielle 23 conformée pour coopérer de manière complémentaire avec un rebord 33 du couvercle 3. En outre, selon l'invention, le boitier 2 comprend au moins une deuxième surface d'appui 24, s'étendant dans le volume interne et conformée pour recevoir une portion périphérique 41 de l'organe de filtration 4, ladite deuxième surface d'appui 24 étant conformée pour coopérer avec une portion 34 du couvercle 3, de sorte que la portion périphérique 41 de l'organe de filtration 4 se trouve pincée entre le couvercle 3 et le boitier 2 comme on peut le voir en détail en figure 3. De plus, le boitier 2 comprend une troisième surface d'appui 25 destinée à recevoir la grille de support 6 et plus particulièrement à recevoir un bord périphérique 65 de la grille de support 6. La troisième surface d'appui 25 s'étend dans le volume interne du boitier 2 et correspond à un épaulement externe 26 du boitier 2, comme visible en figure 8.

Comme on peut le voir en figure 8, chaque surface d'appui 23, 24, 25, présente un diamètre différent et avantageusement, la première portion d'appui 23 présente un diamètre supérieur à la deuxième portion d'appui 24 qui elle-même présente un diamètre supérieur à la troisième portion d'appui 25.

Comme on peut le voir nettement en figure 6, le fond 22 du boitier 2 comprend une pluralité de lames de support 27 configurées pour supporter la grille de support 6 et guider le fluide vers le port de sortie de fluide 12. Les lames de support 27 sont agencées radialement par rapport au port de sortie de fluide 12. Les lames de support 27 sont saillantes par rapport à la surface du fond 22 et s'étendent sensiblement perpendiculairement à ladite surface du fond 22, comme visible en coupe transversale illustrée en figure 8.

Comme on peut le voir notamment en figure 8, le fond 22 du boitier 2 comprend une surface 28 s'étendant radialement autour du port de sortie de fluide 12 jusqu'à la paroi latérale 21 du boitier 2, ladite surface interne 28 étant inclinée et convergeant vers le port de sortie de fluide 12, afin de faciliter l'évacuation du fluide. Dans l'exemple illustré aux figures 2 et 8, l'inclinaison β de la surface 28 du fond 22 est strictement inférieure à 0° et est comprise entre -5° et -10°. Des tests ont été réalisés par la Déposante qui a constaté que si le fond était plan, le fluide ne s'évacuait pas ou que partiellement.

Le couvercle 3 du dispositif 1 va maintenant être décrit en détail en référence aux figures 1, 2, 5 et 7.

Comme on peut le voir en figure 1, le couvercle 3 comprend une base de forme sensiblement cylindrique, surmontée du port d'entrée de fluide 11. Le couvercle 3 comprend une surface interne 31 fermant le volume interne du boitier 2. La surface interne du couvercle 3 s'étend radialement autour du port d'entrée de fluide 11 jusque sur un bord périphérique 32 du couvercle 3, ladite surface interne 31 étant inclinée et convergeant vers le port d'entrée de fluide 11. La surface interne 31 présente donc une forme globale tronconique s'étendant depuis le port d'entrée de fluide 11 et s'élargissant jusqu'au bord périphérique 32 du couvercle 3. Dans une variante non représentée, la surface interne peut être incurvée.

Dans l'exemple illustré en figures 2 et 7, l'inclinaison α de la surface interne 31 du couvercle 3 est strictement supérieure à +4° et est comprise entre +5° et +15°. Des tests ont été réalisés par la Déposante qui a constaté que si la surface interne du couvercle présentait une inclinaison inférieure ou égale à 4°, le fluide n'était pas distribué uniformément sur le l'organe de filtration 4.

Comme on peut le voir en figure 7, l'organe de filtration 4 est agencé dans le boitier 2 à une distance déterminée h du port d'entrée de fluide 11. Avantageusement, la distance déterminée h est strictement supérieure à 1mm et est préférentiellement comprise entre 1,5 et 5mm. En effet, des tests conduits par la déposante ont démontré qu'il était nécessaire que l'organe de filtration 4 soit à une distance déterminée h de manière à ce que les microorganismes puissent croitre lorsque dispositif 1 selon l'invention était mis en incubation puissent. Cette distance déterminée h conditionne un volume minimal d'air nécessaire à la croissance desdits microorganismes. Dans l'exemple illustré aux figures 1, 2, 5, et 7, le couvercle 3 est transparent ou translucide, ce qui permet d'observer la croissance des microorganismes.

Selon une caractéristique de l'invention, la grille de support est configurée pour supporter la couche nutritive et l'organe de filtration 4.

La grille de support 6 du dispositif 1 va maintenant être décrite en détail en référence aux figures 2 et 4.

Comme on peut le voir en figure 2, la grille de support 6 est agencée entre la couche nutritive 5 et le fond 22 du boitier 2. La grille de support 6 est de forme globalement cylindrique et de même dimension que la couche nutritive 5.

Comme on peut le voir en figure 4, la grille de support 6 comprend une pluralité d'ajours 61 traversants répartis sur toute la grille 6, ce qui permet de distribuer le fluide ayant traversé la couche nutritive 5 sur toute la surface 28 du fond 22 afin de mieux l'évacuer rapidement.

Comme illustré en figure 2, la grille de support 6 repose sur les lames de support 27 du boitier 2. La grille de support 6 comprend un bord périphérique 65 reposant sur la troisième surface d'appui 25 du boitier 2 comme visible en figure 2, et 3.

Dans une variante non représentée, la grille ne repose que sur la troisième surface d'appui 25 du boitier, les lames de support étant optionnelles.

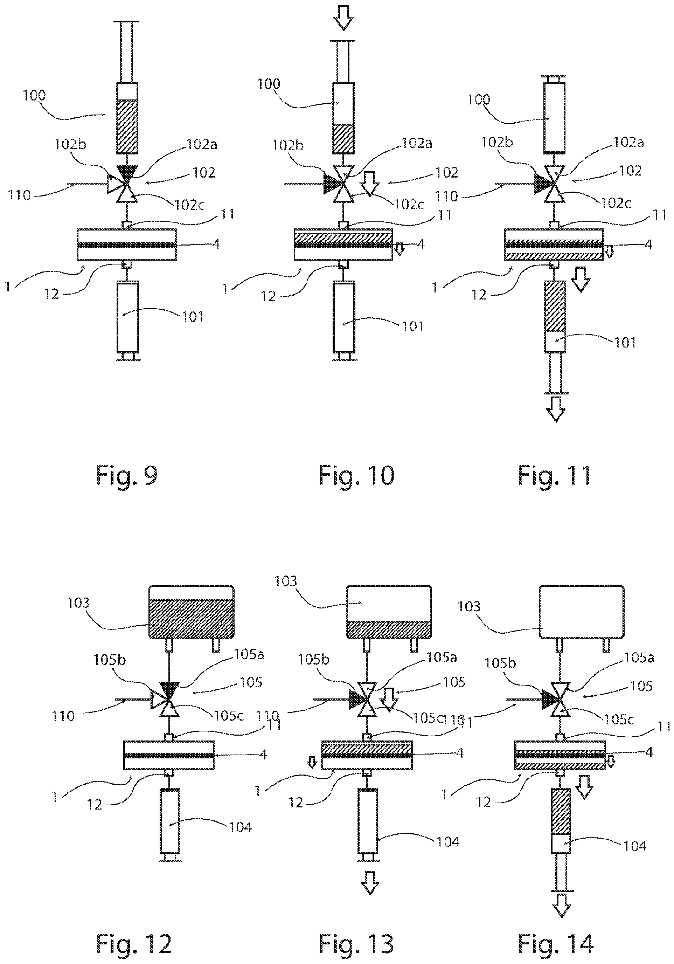

Deux utilisations possibles du dispositif selon l'invention vont maintenant être décrites en référence aux figures 9 à 14.

Selon une première utilisation illustrée aux figures 9 à 11, on connecte au port d'entrée de fluide 11 du dispositif 1, une seringue 100 ou tout autre élément comportant un piston contenant le fluide à analyser, et on connecte au port de sortie de fluide 12 du dispositif 1, un collecteur 101 ou une autre seringue, comme illustré en figure 9. Une vanne trois voies 102 est positionnée entre le dispositif 1 et la seringue 100, la seringue 100 étant connectée indirectement au port d'entrée de fluide 11 du dispositif 1. Initialement et comme illustré en figure 9, la première entrée 102a de la vanne 102, connectée à la seringue 100 est fermée, la deuxième entrée 102b est ouverte et connectée à une arrivée d'air, et la sortie 102c est également ouverte et connectée au port d'entrée de fluide 11 du dispositif. Par ce biais, il est possible de faire le vide avant utilisation du dispositif et avant introduction du fluide à analyser/filtrer.

Puis, comme visible en figure 10, on ouvre la première entrée 102a de la vanne 102 et on introduit le fluide par injection dans le dispositif 1, le piston de la seringue 100 permettant de pousser le fluide dans le dispositif 1, la deuxième entrée 102b de la vanne est alors close et la sortie 102c est ouverte afin de laisser le fluide s'écouler à travers le dispositif 1.

Une fois l'ensemble du fluide à analyser injecté à travers le dispositif 1, il est récupéré dans le collecteur 101, comme visible en figure 11.

Selon une deuxième utilisation illustrée aux figures 12 à 14, on connecte au port d'entrée de fluide 11 du dispositif 1, un réservoir 103 contenant le fluide à analyser, et on connecte au port de sortie de fluide 12 du dispositif 1, une seringue 104 ou tout autre élément à piston permettant l'aspiration du fluide, comme illustré en figure 12. Une vanne trois voies 105 est positionnée entre le dispositif 1 et la seringue 100, la seringue 100 étant connectée indirectement au port d'entrée de fluide 11 du dispositif 1. Initialement et comme illustré en figure 12, la première entrée 105a de la vanne 102, connectée au collecteur 103 est fermée, la deuxième entrée 105b est ouverte et connectée à une arrivée d'air, et la sortie 105c est également ouverte et connectée au port d'entrée de fluide 11 du dispositif 1.

Puis, comme visible en figure 13, on ouvre la première entrée 105a de la vanne 105 et on aspire le fluide contenu dans le collecteur 103 par la seringue 104 positionnée en aval du dispositif 1, la deuxième entrée 105b de la vanne est alors close et la sortie 105c est ouverte afin de laisser le fluide s'écouler à travers le dispositif 1.

Une fois l'ensemble du fluide à analyser aspiré à travers le dispositif 1, il est récupéré dans la seringue 104, comme visible en figure 14. Bien entendu, les seringues et réservoirs ou collecteurs présentent une capacité adaptée au volume de fluide à analyser.

Quelle que soit l'utilisation du dispositif décrite ci-avant, une fois l'injection du fluide terminée (Figures 11 et 14), il est avantageux d'injecter de l'air stérile via l'arrivée d'air 110 pour éliminer toute eau stagnante de la surface de l'organe de filtration 4 du dispositif 1.

En effet, la présence de fluide, même en petites quantités, à la surface de l'organe de filtration 4 pourrait en effet conduire à la diffusion de colonies bactériennes, à de faux résultats négatifs ou à certaines difficultés pour compter les colonies après l'étape d'incubation. À cette fin, de l'air stérile a été aspiré par la seringue 100 ou le réservoir 103 à travers la vanne 102, 105, puis injecté à l'intérieur du dispositif 1, afin de sécher la surface de l'organe de filtration 4. Les avantages de ces utilisations sont le fait que ces techniques d'utilisation sont simples, réalisables sans effort notamment la première utilisation, le flux de travail est d'une durée total inférieure à 1 minute, aucun besoin d'une infrastructure de laboratoire pour effectuer ces manipulations, aucun besoin d'une main d'œuvre qualifiée en microbiologie, une très bonne reproductibilité de l'opération.

Bien entendu, l'invention n'est pas limitée aux modes de réalisation décrits et/ou représentés aux figures annexées. Des modifications restent possibles, notamment du point de vue de la constitution des divers éléments ou par substitution, sans sortir pour autant du domaine de protection de l'invention qui est défini par les revendications ci-jointes.

## Revendications

1. Dispositif de détermination d'une contamination de microorganismes d'un fluide (1), ledit dispositif (1) comprenant :
- un boitier (2) comprenant un volume interne délimité par au moins une paroi latérale (21) et un fond (22),
- un couvercle (3) fermant le boitier (2) et positionné en regard du fond (22),
- un port d'entrée de fluide (11) agencé sur le couvercle (3) et débouchant dans le volume interne dudit boitier (2), le port d'entrée de fluide (11) s'étendant selon un axe sensiblement sécant et préférentiellement perpendiculaire au couvercle (3),
- au moins un organe de filtration (4), agencé dans le volume interne,
- au moins une couche nutritive (5) comprenant une composition d'un milieu de culture microbiologique,
- un port de sortie de fluide (12) ménagé sur le boitier (2) et débouchant dans le volume interne dudit boitier (2),
le dispositif (1) étant **caractérisé en ce que** :
- le couvercle (3) comprend une surface interne (31) dans le volume interne s'étendant radialement autour du port d'entrée de fluide (11) jusque sur un bord périphérique du couvercle (3), ladite surface interne (31) étant inclinée ou incurvée et convergeant vers le port d'entrée de fluide (11),
- le dispositif comprend une grille de support (6) configurée pour supporter la couche nutritive (5) et
- le fond (22) du boitier (2) comprend une surface (28) s'étendant radialement autour du port de sortie de fluide (12) jusqu'à la paroi latérale (21) du boitier (2), ladite surface (28) étant inclinée et convergeant vers le port de sortie de fluide (12).

2. Dispositif selon la revendication 1, dans lequel l'inclinaison de la surface interne (31) du couvercle (3) est strictement supérieure à +4°, l'inclinaison de la surface interne (31) du couvercle (3) est comprise préférentiellement entre +5° et +15° et encore plus préférentiellement égale à sensiblement 10°.

3. Dispositif selon l'une quelconque des revendications 1 ou 2, dans lequel l'inclinaison de la surface (28) du fond (22) est strictement inférieure à 0°, l'inclinaison de la surface du fond est comprise préférentiellement entre -5° et -10° et encore plus préférentiellement entre -6,5° et - 7,5°.

4. Dispositif selon l'une quelconque des revendications 1 à 3, dans lequel le port d'entrée de fluide (11) est saillant dans le volume interne du boitier (2) par rapport à la surface interne (31) du couvercle (3).

5. Dispositif selon l'une quelconque des revendications 1 à 4, dans lequel l'organe de filtration (4) est agencé dans le boitier (2) à une distance déterminée h du port d'entrée (11), la distance déterminée h étant strictement supérieure à 1mm.

6. Dispositif selon l'une quelconque des revendications 1 à 5, dans lequel le boitier (2) comprend une première surface d'appui (23) circonférentielle conformée pour coopérer de manière complémentaire avec un rebord (33) du couvercle (3).

7. Dispositif selon l'une quelconque des revendications 1 à 6, dans lequel le boitier (2) comprend au moins une deuxième surface d'appui (24), s'étendant dans le volume interne et conformée pour recevoir une portion périphérique (41) de l'organe de filtration (4), ladite deuxième surface d'appui (24) étant conformée pour coopérer avec une portion (34) du couvercle (3), de sorte que la portion périphérique (41) de l'organe de filtration (4) se trouve pincée entre le couvercle (3) et le boitier (2).

8. Dispositif selon l'une quelconque des revendications 1 à 7, dans lequel le fond (22) du boitier (2) comprend des lames de support (27) agencées radialement par rapport au port de sortie de fluide (12), lesdites lames (27) étant configurées pour soutenir la grille de support (6) et guider le fluide vers le port de sortie de fluide (12).

9. Ensemble comprenant au moins une amenée de fluide, et au moins un dispositif (1) selon l'une quelconque des revendications précédentes, ledit dispositif (1) étant raccordé par son port d'entrée (11) de fluide à une amenée de fluide.

## Patentansprüche

1. Vorrichtung zur Bestimmung einer Kontamination eines Fluids (1) durch Mikroorganismen, wobei die Vorrichtung (1) Folgendes umfasst:
- ein Gehäuse (2), das ein Innenvolumen umfasst, das durch mindestens eine Seitenwand (21) und einen Boden (22) begrenzt ist,
- eine Abdeckung (3), die das Gehäuse (2) verschließt und gegenüber dem Boden (22) angeordnet ist,
- eine Fluideinlassöffnung (11), die auf der Abdeckung (3) angeordnet ist und in das Innenvolumen des Gehäuses (2) einmündet, wobei sich die Fluideinlassöffnung (11) entlang einer Achse erstreckt, die sie im Wesentlichen schneidet und vorzugsweise senkrecht zur Abdeckung (3) ist,
- mindestens ein Filtrationselement (4), das im Innenvolumen angeordnet ist,
- mindestens eine Nährstoffschicht (5), die eine Zusammensetzung eines mikrobiologischen Kulturmediums umfasst,
- eine Fluidauslassöffnung (12), die auf dem Gehäuse (2) ausgebildet ist und in das Innenvolumen des Gehäuses (2) einmündet,
wobei die Vorrichtung (1) **dadurch gekennzeichnet ist, dass**:
- die Abdeckung (3) eine Innenfläche (31) im Innenvolumen umfasst, die sich rund um die Fluideinlassöffnung (11) bis zu einem Umfangsrand der Abdeckung (3) radial erstreckt, wobei die Innenfläche (31) geneigt oder gekrümmt ist und in Richtung der Fluideinlassöffnung (11) verläuft,
wobei die Vorrichtung Folgendes umfasst:
- ein Stützgitter (6), das so ausgebildet ist, dass es die Nährstoffschicht (5) abstützt,
und
- der Boden (22) des Gehäuses (2) eine Fläche (28) umfasst, die sich rund um die Fluidauslassöffnung (12) bis zur Seitenwand (21) des Gehäuses (2) radial erstreckt, wobei die Fläche (28) geneigt ist und in Richtung der Fluidauslassöffnung (12) verläuft.

2. Vorrichtung nach Anspruch 1, wobei die Neigung der Innenfläche (31) der Abdeckung (3) streng größer als +4° ist, die Neigung der Innenfläche (31) der Abdeckung (3) vorzugsweise zwischen +5° und +15° beträgt und noch mehr bevorzugt im Wesentlichen gleich 10° ist.

3. Vorrichtung nach einem der Ansprüche 1 oder 2, wobei die Neigung der Fläche (28) des Bodens (22) streng kleiner als 0° ist, die Neigung der Fläche des Bodens vorzugsweise zwischen -5° und -10° beträgt und noch mehr bevorzugt zwischen -6,5° und -7,5° beträgt.

4. Vorrichtung nach einem der Ansprüche 1 bis 3, wobei die Fluideinlassöffnung (11) in Bezug auf die Innenfläche (31) der Abdeckung (3) in das Innenvolumen des Gehäuses (2) ragt.

5. Vorrichtung nach einem der Ansprüche 1 bis 4, wobei das Filtrationselement (4) in einem gegebenen Abstand h von der Einlassöffnung (11) im Gehäuse (2) angeordnet ist, wobei der gegebene Abstand h strikt größer als 1 mm ist.

6. Vorrichtung nach einem der Ansprüche 1 bis 5, wobei das Gehäuse (2) eine erste umlaufende Auflagefläche (23) umfasst, die so ausgestaltet ist, dass sie mit einem Rand (33) der Abdeckung (3) komplementär zusammenwirkt.

7. Vorrichtung nach einem der Ansprüche 1 bis 6, wobei das Gehäuse (2) mindestens eine zweite Auflagefläche (24) umfasst, die sich im Innenvolumen erstreckt und so ausgestaltet ist, dass sie einen Umfangsabschnitt (41) des Filtrationselements (4) aufnimmt, wobei die zweite Auflagefläche (24) so ausgebildet ist, dass sie mit einem Abschnitt (34) der Abdeckung (3) zusammenwirkt, sodass der Umfangsabschnitt (41) des Filtrationselements (4) zwischen der Abdeckung (3) und dem Gehäuse (2) eingeklemmt ist.

8. Vorrichtung nach einem der Ansprüche 1 bis 7, wobei der Boden (22) des Gehäuses (2) Stützrippen (27) umfasst, die in Bezug auf die Fluidauslassöffnung (12) radial angeordnet sind, wobei die Rippen (27) so ausgebildet sind, dass sie das Stützgitter (6) abstützen und das Fluid in Richtung der Fluidauslassöffnung (12) leiten.

9. Anordnung, die mindestens eine Fluidzufuhr und mindestens eine Vorrichtung (1) nach einem der vorhergehenden Ansprüche umfasst, wobei die Vorrichtung (1) über ihre Fluideinlassöffnung (11) mit einer Fluidzufuhr verbunden ist.

## Claims

1. Device (1) for determining contamination of a fluid by a microorganism, said device (1) comprising:
- a housing (2) having an internal volume delimited by at least one side wall (21) and a bottom (22),
- a cover (3) which closes the housing (2) and which is positioned opposite the bottom (22),
- a fluid inlet port (11) which is arranged on the cover (3) and which opens out into the internal volume of said housing (2), the fluid inlet port (11) extending along an axis that is substantially secant and preferably perpendicular to the cover (3),
- at least one filtration member (4), arranged in the internal volume,
- at least one nutrient layer (5) comprising a microbiological culture medium,
- a fluid outlet port (12) which is provided on the housing (2) and which opens out into the internal volume of said housing (2),
the device (1) being **characterized in that**:
- the cover (3) has an inner surface (31) in the internal volume extending radially around the fluid inlet port (11) up to a peripheral edge of the cover (3), said inner surface (31) being inclined or curved and converging towards the fluid inlet port (11),
- the device comprises a support grille (6) configured to support the nutrient layer (5), and
- the bottom (22) of the housing (2) has a surface (28) extending radially around the fluid outlet port (12) up to the side wall (21) of the housing (2), said surface (28) being inclined and converging towards the fluid outlet port (12).

2. Device according to Claim 1, in which the inclination of the inner surface (31) of the cover (3) is strictly greater than +4°, the inclination of the inner surface (31) of the cover (3) is preferably between +5° and +15°, and still more preferably equal to substantially 10°.

3. Device according to either one of Claims 1 and 2, in which the inclination of the surface (28) of the bottom (22) is strictly less than 0°, the inclination of the surface of the bottom is preferably between -5° and -10°, and still more preferably between -6.5° and -7.5°.

4. Device according to any one of Claims 1 to 3, in which the fluid inlet port (11) projects into the internal volume of the housing (2) in relation to the inner surface (31) of the cover (3).

5. Device according to any one of Claims 1 to 4, in which the filtration member (4) is arranged in the housing (2) at a given distance h from the inlet port (11), the given distance h being strictly greater than 1 mm.

6. Device according to any one of Claims 1 to 5, in which the housing (2) has a first circumferential bearing surface (23) shaped to cooperate complementarily with an edge (33) of the cover (3).

7. Device according to any one of Claims 1 to 6, in which the housing (2) has at least one second bearing surface (24) which extends in the internal volume and which is shaped to receive a peripheral portion (41) of the filtration member (4), said second bearing surface (24) being shaped to cooperate with a portion (34) of the cover (3) such that the peripheral portion (41) of the filtration member (4) is clamped between the cover (3) and the housing (2).

8. Device according to any one of Claims 1 to 7, in which the bottom (22) of the housing (2) has supporting ribs (27) arranged radially in relation to the fluid outlet port (12), said ribs (27) being configured to hold the support grille (6) and to guide the fluid towards the fluid outlet port (12).

9. Assembly comprising at least one fluid feed and at least one device (1) according to any one of the preceding claims, said device (1) being connected by means of the fluid inlet port (11) thereof to a fluid feed.
